(19) Europäisches Patentamt
European Patent Office
Office européen des brevets

(11) Publication number: 0 199 042 A1

(12) **EUROPEAN PATENT APPLICATION**

(21) Application number: 86103161.5

(22) Date of filing: 10.03.86

(51) Int. Cl.⁴: G01N 33/58 , G01N 33/533 , C07D 493/10 , C07K 15/00

(30) Priority: 26.03.85 US 716015

(43) Date of publication of application: 29.10.86 Bulletin 86/44

(84) Designated Contracting States: BE DE FR IT

(71) Applicant: ABBOTT LABORATORIES
14th Street and Sheridan Road North St
North Chicago Illinois 60064(US)

(72) Inventor: Heiman, Daniel Feulner
407 Drake
Libertyville Illinois 60048(US)
Inventor: Yang Hu, Hsiang-Yun
305 Walker Place
Mundelein Illinois 60060(US)
Inventor: Flentge, Charles Arthur
25296 W. Wayside Place
Lake Villa Illinois 60046(US)

(74) Representative: Modiano, Guido et al
MODIANO, JOSIF, PISANTY & STAUB
Modiano & Associati Via Meravigli, 16
I-20123 Milan(IT)

(54) **Procainamide assay, tracers, immunogens and antibodies.**

(57) The present invention is directed to a fluorescence polarization immunoassay for procainamide, to the various components needed for preparing and carrying out such an assay, and to methods of making these components. Specifically, tracers, immunogens and antibodies are disclosed, as well as methods for making them. The tracers and the immunogens are made from analogs of procainamide. A fluorescein moiety is included in the tracer while a poly(amino acid) forms a part of the immunogen. The assay is conducted by measuring the degree of polarization retention of plane polarized light that has been passed through a sample containing antiserum and tracer.

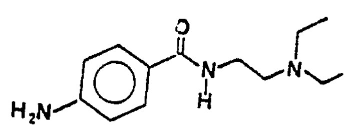

Fig. 1

EP 0 199 042 A1

# PROCAINAMIDE ASSAY, TRACERS, IMMUNOGENS AND ANTIBODIES

## BACKGROUND OF THE INVENTION

### 1. Technical Field

The present invention relates to a method and reagents for a fluorescence polarization immunoassay procedure for determining the amount of procainamide in fluids, especially biological fluids such as serum, plasma or urine, and to a method of making the reagents. More specifically, the invention relates to (1) reagents (tracers and antibodies) for determining the amount of procainamide in a sample; (2) immunogen compounds used to raise antibodies; (3) synthetic methods (for making the tracer and immunogen compounds); and (4) analytical methods for conducting the assay.

### 2. Background Art

Procainamide is an effective and widely prescribed antiarrhythmic drug. It has narrow therapeutic index and can produce serious toxic side effects such as bradycardia, hypotension, arrhythmias, disorientation, malaise, nausea, vomiting and drug-induced systemic lupus erythematosus. The relationship between the dosage of this drug which is administered and the resulting plasma concentration of procainamide has been found to be quite variable among patients. This variability is due to several factors: 1) Differences in drug metabolism among individuals; 2) Changes in the condition of the individual patient during therapy that alter drug metabolism; and 3) Pathologic conditions, such as renal impairment or cardiac failure, that have a profound effect on drug dispoistion. Therefore, monitoring the procainamide level in patient serum or plasma is recommended for safe and effective therapy.

In the past, patient serum or plasma procainamide levels have typically been measured by high performance liquid chromatography (HPLC), enzyme immunoassay (EIA) and substrate-linked fluorescence immunoassay (SLFIA). These methods are reasonably specific for detecting drug levels, however, they are not without drawbacks. HPLC methods require sample extraction procedures and the assay time is lengthy. Both EIA and SLFIA involve enzyme reactions and have the following disadvantages: 1) Reagents are relatively unstable; 2) Any components in the biological samples which may influence the enzyme reaction in EIA or SLFIA (such as enzyme inhibitors or enzymes which catalyze similar reactions) will affect the assay results; and 3) EIA and SLFIA measure either absorbance or fluorescence, and any compounds in the biological samples which may affect absorbance or fluorescence (such as lipid, hemoglobin, bilirubin or other chromophores or fluorophores) will affect the accuracy of the results obtained from these assays.

In assays for other substances, competitive binding immunoassays have provided a more satisfactory alternative. Typically, competitive binding immunoassays are used for measuring ligand in a test sample. (For purposes of this disclosure, a "ligand" is a substance of biological interest to be determined quantitatively by a competitive binding immunoassay technique). The ligands compete with a labeled reagent, or "ligand analog," or "tracer," for a limited number of receptor binding sites on antibodies specific to the ligand and ligand analog. The concentration of ligan in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantative means for measuring the amount of tracer-antibody conjugate produced in a competitive binding immunoassay. Fluorescence polarization techniques are based on the principle that a fluorescent labelled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than the corresponding tracer-antibody conjugate and the molecules are more randomly oriented. As a result, the light emitted from the unbound tracer molecules is depolarized.

Such fluorescence polarization techniques have been applied in U.S. Patent No. 4,420,568 to Wang, et al., which is directed to the use of a triazinylamino-fluorescein moiety as the fluorophore. Procainamide antigen conjugates and antibodies are described in U.S. Patent No. 4,235,969 to Singh, et al. Also, aniline-derived antibodies are described in J. Pharm. Sci., 69 pp. 721-24 (1980) and in Journal of Immunoassay, 3, pp.

91-110 (1982). The present invention, however, offers an advance in the art, in that novel antigens, methods for preparing these antigens, highly sensitive tracers, methods for making the tracers, and an assay using the tracers are provided specifically for the determination of procainamide. The assay conducted in accordance with the present invention is particularly accurate, as will be explained below.

SUMMARY OF THE INVENTION

The present invention is directed to a fluorescence polarization assay for procainamide; to tracers, immunogens and antibodies for use in the assay; and to methods for making the tracers, immunogens and antibodies.

A first aspect of the invention relates to the discovery of unique tracers and immunogens having novel structures. According to the first aspect of the invention, the tracers and the immunogens can both be represented by the structural formula shown in Figure 5 wherein:

Q is a poly(amino acid), a poly(amino acid) derivative, fluorescein, or a fluorescein derivative;

X is NH, C=NH, C=S, CO or $SO_2$;

n is 0, 1 or 2;

R is a linking group including up to 5 heteroatoms when Q is poly(amino acid) or a poly(amino acid) derivative or other immunologically active carrier and up to 8 heteroatoms when Q is fluorescein or a fluorescein derivative, and having a total of from 0 to 18 carbon atoms and heteroatoms;

R' is an alkyl radical having from 1 to 3 carbon atoms;

Y is OH, $NH_2$, $CH_3$, F, Cl, Br, $CF_3$ or H when Q is fluorescein or a fluorescein derivative, and Y is H, OH, $CH_3$, F or Cl when Q is a poly(amino acid) or a poly(amino acid) derivative; and

Z is H or F when Q is fluorescein or a fluorescein derivative, and is H when Q is a poly(amino acid) or a poly(amino acid) derivative or other immunologically active carrier, and Z is attached to any of the carbon atoms of the benzene ring other than those attached to Y or the carboxamide group.

When Q is a poly(amino acid) or a derivative thereof, the compound can be used as an immunogen. When Q is a fluorescein or a derivative thereof, the compound can be used as a tracer.

A second aspect of the invention relates to antibodies raised by the novel immunogen. According to the second aspect of the invention, antibodies are prepared in response to a compound according to Claim 1 when Q is a poly(amino acid) or a derivative thereof or other immunologically active carrier.

According to a third aspect of the invention, an immunogen is made by a method comprising the step of coupling a compound represented by the structural formula shown in Figure 2, wherein:

R is a linking group including up to 6 heteroatoms and having a total of from 0 to 18 carbon atoms and heteroatoms;

R' is an alkyl radical having from 1 to 3 carbon atoms;

X is $NH_2$, COOH, CN, CHO, $SO_3H$, Br, I or OH;

and

Y is H, OH, $CH_3$, F or Cl;

with a poly(amino acid) or a derivative of a poly(amino acid) or other immunologically active carrier.

According to a fourth aspect of the invention, a method is provided for making a tracer by coupling a compound represented by the structural formula shown in Figure 3, wherein:

R is a linking group including up to 8 heteroatoms and having a total of from 0 to 18 carbon atoms and heteroatoms;

R' is an alkyl radical having from 1 to 3 carbon atoms;

X is $NH_2$, COOH, CN, $SO_3H$ or OH;

Y is OH $NH_2$, $CH_3$ F, Cl, Br, $CF_3$ or H; and

Z is H or F and is attached to any of the carbon atoms of the benzene ring other than those attached to Y or to the carboxamide group;

with fluorescein or a derivative of fluorescein.

According to a fifth aspect of the invention, a process for measuring concentration of procainamide is provided. A sample is contacted with procainamide antiserum, and a fluorescein-containing procainamide derivative capable of producing a detectable fluorescence polarization response to the presence of the procainamide antiserum. Plain

polarized light is then passed through the solution to obtain a fluorescence polarization response, and this response is detected as a measure of the amount of procainamide in the sample.

Further aspects and attendant advantages of the invention will be best understood from a reading of the following detailed description taken together with the drawings.

BRIEF DESCRIPTION OF THE DRAWINGS

In the following Figures the symbol "F1" representa a fluorescein moiety, and the various other symbols are noted in the Detailed Description. It should be noted that in Figures 3, 5, 7 and 10, the group other than "Y" and the carboxamide substituent can be located at any position of the benzene ring not occupied by the "Y" and the carboxamide substituents.

Figure 1 shows the structure of the drug procainamide, which can be quantitatively determined in accordance with the present invention.

Figure 2 shows a class of reactants for a method of making an immunogen in accordance with the present invention.

Figure 3 shows a class of reactants for a method of making a tracer in accordance with the present invention.

Figure 4 shows the alternate structural formulae and names of the fluorescein moiety included in the tracers of the present invention.

Figure 5 shows a general structural formula for the tracers and the immunogens of the present invention.

Figure 6 shows a general structural formula for the immunogens of the present invention.

Figure 7 shows a general structural formula for the tracers of the present invention.

Figure 8 shows a structural formula for preferred immunogens of the present invention.

Figure 9 shows a structural formula for preferred tracers of the present invention.

Figue 10 shows a precursor for the immunogens shown in Figures 6 and 8 and for the tracers shown in Figures 7 and 9.

Figure 11 shows various linkages that couple the fluorescein moiety to the precursor at the X position in Figure 10, when Figure 10 represents the precursor for the tracers shown in Figures 7 and 9.

Figure 12 through 29 show various examples of stuctures of tracers in accordance with the present invention.

Figure 30 through 35 show various examples of structures of hapten reactants which can be used to form the immunogens of the present invention.

DETAILED DESCRIPTION OF THE PREFERRED EMBODIMENTS

The various aspects of the invention will now be discussed in detail and in relation to the drawing Figures.

The present invention involves the use of fluorescein and derivatives of fluorescein. In particular, a necessary property of fluorescein and its derivatives for the usefulness of the tracer compounds is the fluorescence of fluorescein. Fluorescein exists in two tautomeric forms, illustrated in Figure 4, depending on the acid concentration (pH) of the environment. In the open - (acid) form, there are a number of conjugated double bonds which make that form of fluorescein (and compounds containing a fluorescein moiety) capable of absorbing blue light and emitting green fluorescence after an excited state lifetime of about 4 nanoseconds. When the open and closed forms coexist, the relative concentration of molecules in the open and closed forms is easily altered by adjustment of the pH level. Generally, the tracer compounds of the present invention exist in solution as biologically acceptable salts such as sodium potassium ammonium and the like, which allows the compounds to exist in the open, fluorescent form, when employed in the analytical methods of the present invention. The specific salt present will depend on the buffer employed to adjust the pH level. For example, in the presence of an alkaline sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is number 6. In the open form, the para-carbon to the carboxylic acid group on the phenyl ring is numbered 5 (See Figure 4). In this disclosure the numbering of the closed form is adopted because the raw materials used in the syntheses are most popularly numbered with that system. The carbon atom of fluorescein and its compounds which is opposite the carboxyl group is therefore numered 6 for the purposes of the present disclosure.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization obseved. Therefore, when a ligand competes with the tracer for antibody sites, the observed polarization of fluorescence of the resulting mixture of free tracer and tracer-antibody complex assumes a value intermediate between that of the tracer and that of the tracer-antibody complex. If a sample contains a high concentration of the ligand, the observed polarization value is closer to that of the free ligand, i.e., low. If the test samples contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be interpolated from a standard curve prepared in this manner.

The particular antibodies and tracers formed in accordance with this invention have been found to produce surprisingly good assays, as discussed infra.

1. The Reagents

Both the immunogens and the tracers of the present invention can be represented by the general structural formula set forth in the Summary of the Invention, and illustrated in Figure 5. When Q is a poly(amino acid), the structure represents the immunogen; when Q is a fluorescein moiety, the structure represents the tracer.

The objective is to have competition between procainamide and the tracer for the recognition sites of the antibody. Great variations in the structure of the haptens and tracers are allowed in achieving this goal. For the purposes of this invention, "haptens" are precursors of the immunogens, comprising generally a substituted procainamide analog and a linking group to an immunologically active carrier.

a. The Structure of the Immunogens

Usable antibodies can be produced from a variety of procainamide analogs. Immunogens made from compounds that have H, OH, $CH_3$, F or Cl in place of the $-NH_2$ on procainamide can produce antibodies in animals; such antibodies are useful in a procainamide assay according to the invention when combined with the appropriate tracer.

The immunogens of the present invention have the general structural formula shown in Figure 6, and in the preferred formula shown in Figure 6, and in the preferred form of the invention, the immunogens have the structural formula shown in Figure 8. The immunogens can be prepared by coupling a compound of the class shown in Figure 2 with a poly(amino acid) or a derivative of a poly(amino acid) or other immunologically active carrier substance, as will be discussed in the context of the synthetic method and the Examples below.

In a preferred form of the invention, the immunogen has the structural formula shown in Figure 8. This structure is preferred because the best recognition of procainamide occurs when the hapten bears the same nitrogen substituent as the drug to be measured. Although bovine serum albumin is the poly(amino acid) in this preferred

form, it should be understood that various protein carriers may be employed, including albumins, serum proteins, e.g., globulins, ocular lens proteins, lipoproteins and the like. Illustrative protein carriers include bovine serum albumin, keyhole limpet hemocyanin, egg ovalbumin, bovine gamma-globulin, thryoxine binding globulin, etc. Alternatively, synthetic poly(amino acids) having a sufficient number of available amino groups such as lysines may be employed, as may other synthetic or natural polymeric materials bearing reactive functional groups. In particular, carbohydrates, yeasts, or polysaccharides may be conjugated to the hapten to produce an immunogen.

b. The Structure of the Tracers

The possible variations in the structure of the tracers of the invention are even greater than the possible variations in the structure of the haptens thereof. The tracers of the present invention have the general structural formula shown in Figure 7, where F1 represents a fluorescein moiety or a fluorescein derivative. In a preferred form of the invention, the traces have the structural formula shown in Figure 9.

The tracer is a procainamide analog that is linked to a fluorescein derivative by, for example, an amido, amidino, triazinylamino, carbamido, thiocarbamido, carbamoyl, thiocarbamoyl, or sulfonylcarbamoyl group, as shown in Figure 11. The tracers are prepared by linking the appropriate fluorescein derivative to a procainamide analog containing an amino, carboxylic acid, hydroxy, imidate, hydrazide isocyanate, thioisocyanate, chloroformate, chlorothioformate, chlorosulfonylcarbamoyl, or the like group, as will be discussed in the context of the synthetic method and the Examples below.

By way of example, any of the following fluorescein derivatives can be used:

| | |
|---|---|
| $Fl-NH_2$ | fluorescein amine |
| $Fl-CO_2H$ | carboxyfluorescein |
| $Fl-NHCOCH_2I$ | α-iodoacetamidofluorescein |
| $Fl-NHCOCH_2Br$ | α-bromoacetamidofluorescein |

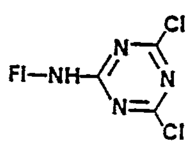

(DTAF) 2,4-dichloro-1,3,5-triazin-2-yl amino-fluorescein

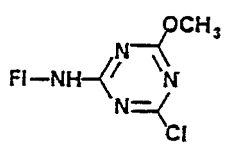

4-chloro-6-methoxy-1,3,5-triazin-2-ylamino fluorescein

Fl-NCS fluorescein thioisocyanate

2. The Antibodies

The antibodies of the present invention are prepared by developing a response in animals to the immunogens described supra. It should be understood that although rabbits are the presently preferred host for the immune response to procainamide immunogens, any host capable of producing antibodies to the immunogen structures herein outlined may be employed, as well as any in vitro solution containing immuno-competent cells. The immunogens are administered to animals or to immuno-competent cell cultures by a series of innoculations, in a manner well-known to those skilled in the art.

3. Synthetic Methods

Both the immunogens and the tracers of the present invention can be made from a precursor having the general structural formula shown in Figure 10, wherein

Y is H, OH, $NH_2$, $CH_3$, F, Cl, Br, $CF_3$ or H when the preparation is directed to a tracer, and Y is H, OH, $CH_3$, F or Cl when the preparation is directed to an immunogen;

X is $NH_2$, COOH, CHO, $SO_3H$, CN, Br I or OH when the preparation is directed to an immunogen, and $NH_2$, COOH CN, $SO_3H$ or OH when the preparation is directed to a tracer; adn

Z is H or F when the preparation is directed to a tracer, and Z is H when the preparation is directed to an immunogen, and wherein Z is attached to any of the carbon atoms of the benzene ring other than those attached to Y or to the carboxamide group.

a. The Synthesis of the Immunogens

The immunogens of the present invention are made by coupling a hapten, such as that shown by the general structure of Figure 2 when X is $NH_2$, COOH, CN, $SO_3H$, CHO, bromine, iodine or OH, to a poly(amino acid) or other immunologically active carrier. The poly(amino acid) or other carrier moiety can be linked to the hapten by an amide, an amidine, an alkyl, a urea, a thiourea, a sulfonamide, carbamate, or a thiocarbamate linkage. In a preferred embodiment, the poly(amino acid) is bovine serum albumin (BSA), and the hapten can be selected from one of the exemplary structures shown in Figures 30 through 35. These reactants are preferably coupled under conditions normally used to form amide linkages; such conditions are well known to those skilled in the art. It is most preferred that water soluble carbodiimide procedures be used, as these are the most effective for forming the desired amide linkages in this context.

The immunogens are prepared by coupling a hapten that contains an $-NH_2$, $-CO_2H$, $-CONHNH_2$, -CNOR, -CHO, -Br, -I, -NCO, -NCSA, -OCOCl, -OCSCl, $-SO_2Cl$ or $-OCONHSO_2Cl$ group to a poly(amino acid). The $-NH_2$ case can be coupled by activating the carboxylic acid group on the polyamino acid in the presence of the $-NH_2$ group. The activation of the carboxylic acid groups on the poly(amino acid) can be accomplished by mixing the hapten and the poly(amino acid) with 1-ethyl-3-(3-dimethylamino-propyl) carbodiimide (EDC), N, N'-dicyclohexylcarbodiimide (DCC), 1-cyclohexyl-3-(2-morpholinoethyl) carbodiimide methyl-p-toluene sulfonate, or the like. The $-CO_2H$ case is also coupled by the in situ activation method (EDC) or the active ester method, as described below in the tracer synthesis section. The $-CONHNH_2$ case is coupled in the same manner as for the amino case. The -CHO case is coupled to the poly(amino acid) by reductive amination. The poly(amino acid) is mixed with the -CHO hapten and the resulting imine is reduced with sodium borohydride or sodium cyanoborohydride to yield alkylated amines on the poly(amino acid). The -Br and -I cases also produce alkylated amines on the poly(amino acid), but by direct coupling of the alkyl-halide to the amine on the poly(amino acid); however, they also react with phenols to give phenyl ethers and with mercapto groups to give thioethers. The isocyanate (-NCO), isothiocyanate (-NCS), chloroformate (-OCOCl), chlorothioformate (-OCSCl), sulfonyl chloride ($-SO_2Cl$) and carbamoyl sulfonyl chloride ($-OCONHSO_2Cl$) cases produce urea, thiourea, carbamate thiocarbamate, sulfonamide and carbamoyl sulfonamide linkages respectively. This is accomplished by direct coupling of the hapten to the polyamino acid.

The synthesis of the above haptens - (immunogen precursors where Y=H, OH, $CH_3$, F or Cl) are accomplished in very similar ways. Figure 2 shows an immunogen precursor class in accordance with a preferred embodiment of the method the present invention. The ideal starting material, in general, is the methyl ester or acid halide of the appropriate substituted benzoic acid; for example, 4-toluoyl chloride. These activated acids can be coupled with N-ethyl ethylenediamine and alkylated with a variety of reagents that will enable different derivatives to be prepared. Reaction with the appropriate halide or sulfonate ester (for example, ethyl-4-bromocrotonate) followed by reduction, if necessary, and hydrolysis can prepare homologous carboxylic acid derivatives. Reaction with the appropriate chloro-, bromo-or sulfonyloxy-nitrile gives the homologous amines after catalytic reduction. Alcohols and aldehydes are obtainable by alkylation with the appropriate halo-olefin, followed by ozonolysis and reduction under more or less vigorous conditions. Acetal-protected halo-aldehydes can also serve as precursors to procainamide-aldehydes, and homologous alcohols can also be obtained directly by the use of chloro-, bromo-or iodo-alcohols as the alkylating agents. The use of bromoethanesulfonic acid or its congeners give the homologous procainamide sulfonic acids. If the use of a protecting group is required on the substituted benzoic acid (e.g. when Y is OH or $NH_2$), this will normally be removed at a point in the synthetic

sequence after the alkylation has been carried out. The preferred protection for the hydroxyl group is the benzyl ether; for the amine, the preferred method is to carry it through as a nitro group and reduce this to the amine near the end of the synthetic pathway.

The carboxylic acid derivatives are suitable for coupling to produce immunogens. The nitrile derivatives are converted to alkoxy imidates by treating the nitrile with anhydrous alcohol and hydrogen chloride gas. The alkoxy imidates are then suitable for coupling. The hydrazide derivatives are prepared from the corresponding carboxylic acid derivatives by active ester coupling with hydrazine or by reacting hydrazine with the corresponding carboxylic ester derivative. The aldehyde derivatives are also suitable for coupling to produce immunogens. They are also convertible to the corresponding amines as are the nitriles. For the aldehydes, this is accomplished by reductive amination or by the preferred conversion to the corresponding oxime followed by reduction. For convenience, reductions of the olefin, nitrile or oxime and, for $Y=NO_2$, the nitro group, ar affected simultaneously. These amino derivatives are then suitable for coupling to produce immunogens. With the appropriate protection of the Y groups (when necessary), the amine is convertible to an isocyanate or thioisocyanate group by reaction of the amine with phosgene or thiophosgene. The isocyanate and thioisocyanate derivatives are then coupled to a poly(amino acid) and then the protecting groups are removed.

The aldehyde derivatives can also be converted to the corresponding alcohols by reduction. This can be accomplished with sodium borohydride, hydrogenation, or the like. The alcohols can be converted to the bromo or iodo derivatives with phosphorous tribromide, phosphorous triiodide, or the like; also, the iodo derivative can be prepared from the bromo derivative by halogen exchange with sodium iodide or the like. The halo derivatives are then suitable for coupling. The nitrile derivatives can also be prepared from he halo derivatives by reaction with cyanide.

The aldehydes can be condensed with -(aminohydroxy)alkylcarboxylic acids, such as $NH_2OCH_2CO_2H$, to produce substituted oxime derivatives. These are suitable for coupling to a poly(amino acid). The oxime alkyl carboxylic acid derivatives can be partially reduced to the corresponding (aminohydroxy)alkylcarboxylic acid derivatives, which also are suitable for coupling to a poly(amino acid). The same type of condensation and reduction can be accomplished with hydrazine and hydrazine derivatives.

The alcohol derivatives can also be converted to the chloroformate, chlorothioformate and chlorosulfonyl carbamoyl derivatives. This is accomplished by reacting the alcohol, with Y = OH or $NH_2$ protected, with phosgene, thiophosgene or chlorosulfonyl isocyanate. The resulting chloroformates, chlorothioformates or chlorosulfonyl carbamates are coupled to a poly(amino acid), and then the protecting groups are removed.

The sulfonic acids are most readily coupled to poly(amino acids) by converting them to the corresponding sulfonic acid chlorides by treatment with phosphorous pentachloride, or the like.

b. The Synthesis of the Tracers

The tracers of the present invention are made by coupling a fluorescein moiety, or a derivative of fluorescein, to the general structure shown in Figure 10 when X is $NH_2$, COOH, CNOR, $SO_3H$ or OH. The fluorescein moiety can be linked to the amino, carboxyl, imidate, sulfonyl or alkoxy functional group by an amide, an amidine, a urea, a thiourea, a carbamate, a thiocarbamate, a triazinylamino, a sulfonamide or a sulfonylcarbamate linkage, as shown in Figure 11. In the presently preferred embodiment, the fluorescein derivative is 6-aminofluorescein (available from Aldrich Chemical Company, Milwaukee), and this coupled to the precursor shown in Figure 3. The reaction is carried out in dimethylformamide. The procainamide analog is activated by forming a mixed anhydride with isobutyl chloroformate. Other solvents, such as dimethylsulfoxide, and other activating reagents, such as ethyl chloroformate, can be used. Usable tracers can be prepared from a wide variety of procainamide analogs.

All procainamide analogs that have a terminal amino group, such as amino, hydrazinyl, hydrazido or the like, are coupled to carboxyfluorescein by the active ester method or the mixed anhydride method, and coupled to fluorescein isothiocyanate, α-iodoacetamidofluorescein, α-bromoacetamidofluorescein, DTAF or alkoxy DTAF by simply mixing the two materials in solution. The amino group can be converted to the isocyanate and thioisocyanate groups by reaction with phosgene and thiophosgene, respectively. These activated compounds are then condensed with aminofluorescein to produce the tracer. The $Y=OH$ and $NH_2$ groups require a protecting group for the conversion of the $X=NH_2$ group to the isocyanate and the thioisocyanate groups,which is removed after the coupling to fluorescein amine is complete.

All procainamide analogs that have a terminal carboxylic acid group, such as carboxylic acid, (aminohydroxy) alkylcareboxylic acid or the like, are coupled to aminofluorescein by the active ester, carbonyl diimidazole or mixed anhydride methods.

All procainamide analogs that have a terminal hydroxy group can be coupled to fluorescein by reaction with DTAF, α-iodoacetamidofluorescein, α-bromoacetamidofluorescein or fluorescein isothiocyanate in solution. The hydroxy group can be coverted to the chlorosulfonylcarbamoyl, chloroformate and chlorothioformate groups by reaction with chlorosulfonylisocyanate, phosgene and thiophosgene, respectively. These derivatives are then coupled to aminofluorescein in solution to produce the tracer. The Y = OH and $NH_2$ groups require a protecting group for the conversion of the hydroxy group to the chlorosulfonylcarbamoyl, chloroformate and chlorothioformate groups which is removed after the coupling to fluorescein amine is complete.

All procainamide analogs that have a terminal nitrile group are converted to imidates in anhydrous alcohol in the presence of hydrogen chloride gas. The imidate is then coupled to fluorescein amine in solution to prepare the tracer.

The preparation of the various amino, carboxylic acid, hydroxy and nitrile derivatives of the benzoic acid derivatives, with the exception of Y = Br and Z = F, were described above in the immunogen preparation section. The derivatives where Z = F are prepared from the corresponding amino derivatives, by the Balz-Schiemann reaction or variants thereof. The amino derivatives are prepared by reduction of the corresponding nitro derivatives. The nitro derivatives are prepared from known benzene derivatives by known methods.

4. The Assay

The particular tracers and antibodies of the present invention have been found to produce surprisingly good results in fluorescence polarization assays for procainamide. Figure 1 shows the structure of the drug procainamide which can be quantitatively determined in accordance with the present invention. The assay of the present invention provides a more convenient and rapid procainamide assay method than prior art methods, because it requires no specimen treatment before analysis, the reagents are chemically and thermally stable, and the assay system has minimal cross-reactivity to procainamide-like compounds.

In accordance with the analytical methods of the present invention, i.e. the methods of determining procainamide by a fluorescence polarization immunoassay procedure using the tracer compounds and immunogens of the invention, a sample containing or suspected of containing procainamide is intermixed with a biologically acceptable salt of a tracer and an antibody specific to procainamide and to the tracer. The antibody is produced using the immunogen as described above. The procainamide and tracer compete for limited antibody sites, resulting in the formation of complexes. By maintaining constant the concentration of tracer and antibody, the ratio of procainamide-antibody complex to tracer-antibody complex that is formed is directly proportional to the amount of procainamide in the sample. Therefore, upon exciting the mixture with linearly polarized light and measuring the polarization of the fluorescence emitted by a tracer and a tracer-antibody complex, one is able to determine quantitatively the amount of procainamide in the sample.

The results can be quantified in terms of net millipolarization units, span (in millipolarization units) and relative intensity. The measurement of millipolarization units indicates the maximum polarization when a maximum amount of the tracer is bound to the antibody in the absence of any procainamide. The higher the net millipolarization units, the better the binding of the tracer to the antibody. The span is an indication of the difference between the net millipolarizations at the points of the maximum and the mimimum amounts of tracer bound to the antibody. A larger span provides for a better numerical analysis of data. The intensity is a measure of the strength of the signal above background. Thus, a higher intensity will give a more accurate measurement. The intensity is determined at about 0.5 to 2.0 nanomolar for the preferred tracers of the invention, as the sum of the vertically polarized intensity plus twice the horizontally polarized intensity. The intensity can range from a signal from about three times to about thirty times the background noise, depending upon the concentration of the tracer and the other assay variables. For the purposes of the present invention, and intensity of at least eight to ten times that of background noise is preferred.

Tables I through III show the results obtained with various embodiments of the present invention, in terms of span, millipolarization units and intensity. In Table I, bovine serum albumin (BSA) was used as the protein carrier and rabbit was the host species. The data demonstrate that tracers bearing a wide variety of phenyl substituents and having

widely diverse linking arms produce useful procainamide assays with antiserum raised against a fluorine-substituted immunogen. Table II demonstrates that other carriers and other animal hosts also produce acceptable procainamide assays. Table III demonstrates that the tracers of this invention produce good procainamide assays with antiserum raised against haptens bearing a variety of phenyl substituents, including those of the prior art. As seen from the data in Tables I, II and III, an assay produced from an immunogen made from the hapten of Figure 32 used in combination with the tracer of Figure 19 provides excellent results. Accordingly, this combination is presently the most preferred form of the invention. In addition, many other hapten/tracer combinations, in particular those represented by the combinations of Figures 35 and 24, Figures 31 and 24, and Figures 32 and 18 also produced acceptable results and are alternative preferred combinations.

Table I

Fluoro-substituted immunogen on BSA carrier in rabbits

| Hapten | Tracer | Net Polarization* | Span* | Intensity** |
|---|---|---|---|---|
| Fig. 31 | Fig. 14 | 140 | 74 | 21 |
| Fig. 31 | Fig. 25 | 177 | 85 | 8 |
| Fig. 31 | Fig. 28 | 215 | 99 | 7 |
| Fig. 31 | Fig. 29 | 202 | 93 | 14 |
| Fig. 31 | Fig. 24 | 216 | 101 | 14 |
| Fig. 31 | Fig. 13 | 172 | 118 | 17 |
| Fig. 31 | Fig. 18 | 212 | 76 | 8 |
| Fig. 31 | Fig. 27 | 187 | 107 | 20 |
| Fig. 31 | Fig. 20 | 193 | 109 | 18 |
| Fig. 31 | Fig. 21 | 230 | 71 | 19 |
| Fig. 31 | Fig. 12 | 168 | 87 | 15 |
| Fig. 31 | Fig. 26 | 185 | 129 | 14 |
| Fig. 31 | Fig. 19 | 177 | 73 | 14 |
| Fig. 31 | Fig. 15 | 186 | 111 | 11 |
| Fig. 31 | Fig. 16 | 187 | 107 | 19 |
| Fig. 31 | Fig. 22 | 156 | 80 | 12 |

* In millipolarization units
** Expressed as the ratio of net intensity to background noise

Table II

The hapten of Fig. 31 coupled to various carriers and used to raise antibodies in various host species

| Carrier | Species | Tracer | Net Polarization* | Span* | Intensity** |
|---|---|---|---|---|---|
| BSA | Sheep | Fig. 25 | 210 | 91 | 7 |
| BSA | Sheep | Fig. 29 | 182 | 58 | 9 |
| BSA | Sheep | Fig. 21 | 203 | 51 | 19 |
| BSA | Sheep | Fig. 24 | 157 | 88 | 12 |
| BSA | Sheep | Fig. 18 | 213 | 52 | 16 |
| BSA | Sheep | Fig. 20 | 189 | 50 | 18 |
| BSA | Sheep | Fig. 27 | 263 | 125 | 5 |
| BSA | Sheep | Fig. 26 | 201 | 77 | 10 |
| BSA | Sheep | Fig. 16 | 191 | 77 | 19 |
| BSA | Sheep | Fig. 15 | 219 | 68 | 11 |
| BSA | Sheep | Fig. 22 | 161 | 77 | 12 |
| Thyroglobulin | Goat | Fig. 24 | 257 | 140 | 11 |
| Thyroglobulin | Rabbit | Fig. 24 | 264 | 145 | 12 |
| Thyroglobulin | Rabbit | Fig. 26 | 169 | 94 | 10 |
| Thyroglobulin | Rabbit | Fig. 23 | 137 | 76 | 12 |
| Thyroglobulin | Rabbit | Fig. 28 | 187 | 107 | 12 |
| Thyroglobulin | Rabbit | Fig. 29 | 189 | 110 | 20 |

* In millipolarization units
** Expressed as the ratio of net intensity to background noise

Table III

Variation of hapten substituent in rabbits

| Hapten | Tracer | Net Polarization* | Span* | Intensity** |
|---|---|---|---|---|
| Fig. 30 | Fig. 24 | 205 | 108 | 14 |
| Fig. 32 | Fig. 22 | 137 | 87 | 18 |
| Fig. 32 | Fig. 18 | 217 | 139 | 6 |
| Fig. 32 | Fig. 20 | 166 | 113 | 8 |
| Fig. 32 | Fig. 19 | 201 | 154 | 18 |
| Fig. 32 | Fig. 26 | 145 | 92 | 13 |
| Fig. 33*** | Fig. 12 | 169 | 106 | 11 |
| Fig. 33 | Fig. 17 | 196 | 133 | 10 |
| Fig. 33 | Fig. 26 | 165 | 102 | 10 |
| Fig. 33 | Fig. 22 | 136 | 96 | 17 |
| Fig. 33 | Fig. 20 | 204 | 137 | 17 |
| Fig. 33 | Fig. 18 | 194 | 104 | 6 |
| Fig. 33 | Fig. 13 | 125 | 70 | 34 |
| Fig. 33 | Fig. 29 | 211 | 132 | 8 |
| Fig. 33 | Fig. 15 | 148 | 106 | 27 |
| Fig. 33 | Fig. 19 | 160 | 100 | 16 |
| Fig. 34 | Fig. 24 | 218 | 123 | 14 |
| Fig. 35 | Fig. 24 | 212 | 146 | 16 |

* In millipolarization units
** Expressed as the ratio of net intensity to background noise
*** Prior Art Immunogen

The pH at which the method of the present invention is practiced must be sufficient to allow the fluorescein moiety of the tracers to exist in their open form. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular buffer employed is not critical to the present invention, but the tris and phosphate buffers are preferred. The cation portion of the buffer will generally determine the cation portion of the tracer salt in solution.

The preferred method of the improved assay of the present invention will now be discussed in detail. The assay is a "homogeneous assay," which means that the end polarization readings are taken from a solution in which bound tracer is not separated from unbound tracer. This is a distinct

advantage over heterogeneous immunoassay procedures such as those where the bound tracer must be separated from the unbound tracer before a reading can be taken.

The reagents for the fluorescence polarization assay of the present invention comprise antibody specific for procainamide and tracer. Additionally, largely conventional solutions including procainamide pretreatment solution, a dilution buffer, procainamide calibrators and procainamide controls are desirably prepared. Typical solutions of these reagents, some of which are described below, are commercially available in assay "kits" from Abbott Laboratories, Abbott Park, Illinois.

All percentages expressed herein are weight/volume unless otherwise indicated. The tracer formulation presently preferred is 61.5 nanomolar tracer in: 0.1 molar tris buffer at pH 7.5; 0.1% sodium dodecyl sulfate; 0.1% sodium azide; and 0.01% bovine gamma globulin. The antiserum formulation comprises rabbit serum diluted with: 0.1 molar phosphate buffer at pH 7.5; 0.1% sodium azide; 0.01% bovine gamma globulin; and 2% ethylene glycol (volume/volume). The dilution buffer comprises: 0.1 molar sodium phosphate at pH 7.5; 0.1% sodium azide; and 0.01% bovine gamma globulin. The pretreatment formulation comprises: 0.01% bovine gamma globulin; 0.1 molar tris buffer at pH 7.5; 0.1% sodium azide; 0.1% sodium dodecyl sulfate. Procainamide calibrators comprising procainamide and normal human serum at concentrations of 0.0, 1.0, 2.5, 5.0, 10.0 and 20.0 milligrams per liter, with 0.1% sodium azide preservative are useful. Procainamide controls comprising procainamide and normal human serum are provided at concentrations of 2.0, 7.0 and 15.0 milligrams per liter with 0.1% sodium azide as a preservative are also useful.

The preferred procedure is especially designed to be used in conjunction with the Abbott TDx@ Polarization Analyzer available from Abbott Laboratories, Irving, Texas. 50 microliters of serum or plasma are required. The calibrators, controls, or unknown samples are pipetted directly into the sample well of the TDx@ sample cartridge. One of the advantages of this procedure is that the sample does not require any special preparation. If a TDx@ procainamide assay kit is being used with the TDx@ analyzer, the caps from each of the three reagent containers in the kit are removed and placed into designated wells inside the TDx@ analyzer, and the assay procedure from this point is fully automated.

If a manual assay is being performed, then the sample is mixed with the pretreatment solution in dilution buffer and a background reading is taken. The tracer is then mixed with the assay. The antibody is then finally mixed into the test solution. After incubation, a fluorescence polarization reading is taken.

The fluorescence polarization value of each calibrator, control or sample is determined and is printed on the output tape of an instrument such as the Abbott TDx@ polarization analyzer. A standard curve is generated in the instrument plotting the polarization of each calibrator versus its concentration using a nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between about 2 and about 8°C, while the dilution buffer should be stored at ambient temperature. A standard curve and controls should be run every two weeks, with each calibrator and control run in duplicate. Controls should be run with each batch, and all samples can be run in duplicate.

It should be understood that the foregoing detailed description and the following Examples are intended to be illustrative, but not limiting, with respect to the scope of the present invention. Various modifications will become apparent to one skilled in the art, and thus it is intended that the scope of the invention be defined solely by the claims and legal equivalents.

## EXAMPLES

Examples I through LXVI describe experiments that were performed in accordance with the concepts of the present invention. Examples I through VI are directed to preparation of immunogens useful for producing antibody; Examples VII through XLVIII are directed to the synthesis of precursors for immunogens and tracers; and Examples XLIX through LXVI are directed to the preparation of tracers.

Example I: <u>Preparation of an Immunogen from the hapten of Figure 32 by the active ester method.</u>

N-[N'-Ethyl-N'-(3-carboxy-1-propyl)-2-aminoethyl]-p -hydroxybenzamide (5.4 mg) and N-hydroxysuccinimide (2.7 mg) were dissolved in 1.0 mL of dimethylformamide and dicyclohexyl carbodiimide (4.9 mg) was added. The solution was

stirred overnight at room temperature, and the resulting mixture was filtered and added to a solution of 0.28 g of thyroglobulin in 30 mL of sodium carbonate buffer (pH 9.8). Stirring was continued overnight at 0 to 5°C, after which the immunogen was isolated by dialysis and lyophylization.

Example II: Preparation of an Immunogen from the hapten of Figure 31 and bovine serum albumin by the active ester method.

N-[N'-Ethyl-N'-(3-carboxy-1-propyl)-2-aminoethyl]-p -fluorobenzamide (20 mg, 0.07 mmol) was taken up in 200 mL of DMF, and 9 mg (0.08 mmol) of N-hydroxysuccinimide and 17 mg - (0.08 mmol) of dicyclohexylcarbodiimide were added. The mixture was stirred overnight at 0 to 4°C, the dicyclohexylurea filtered off and the filtrate was added to 113 mg. (0.0017 mmol) of bovine serum albumin in 3 mL of sodium carbinate buffer (pH 9.8) at 0°C. The mixture was stirred overnight at 0 to 4°C and then dialysed against 1 liter of dilute ammonium hydroxide (pH 9.4) for six days, changing the buffer daily.

Example III: Preparation of an immunogen from the hapten of Figure 31 and thyroglobulin by the active ester method.

N-[N'-Ethyl-N'-(3-carboxy-1-propyl)-2-aminoethyl]-p -fluorobenzamide (50 mg) in 2.0 mL of 1:1 dioxane-dimethylformamide and N-hydroxysuccinimide (25 mg) and dicylcohexyl-carbodiimide (45 mg) were added. The solution was stirred overnight at room temperature and filtered into a solution of thyroglobulin (300 mg) in 25 mL of pH 8.3 phosphate buffer. After stirring overnight at room temperature, dialysis for 3 days vs. distilled water and lyophyllization yielded the product.

Example IV: Preparation of an immunogen from the hapten of Figure 35 by the water-soluble carbodiimide method.

N-[N'-Ethyl-N'-(3-carboxy-1-propyl)-2-aminoethyl]-p -chlorobenzamide (45 mg) and 30 mg of bovine serum albumin were dissolved in 4 mL distilled water, the pH was adjusted to 5.0 and 45 mg of 1-ethyl-3-(3-dimethylaminopropyl) carbodiimide (EDC) was added to the mixture with stirring. After 1 hour another 45 mg of EDC was added. After a further 4 hours at room temperature, the mixture was dialized against 2 liters of distilled water, changing the water 5 times over a 3-day period.

Example V: Preparation of an immunogen from the hapten of Figure 30 by the water-soluble carbodiimide method.

This immunogen was prepared from 53 mg of N-[N'-ethyl-N'-(3-carboxy-1-propyl)-2-aminoethyl] benzamide and 26 mg of bovine serum albumin by the method of Example III.

Example VI: Preparation of an immunogen from the hapten of Figure 34 by the water soluble carbodiimide method.

This immunogen was prepared from 48 mg of N-[N'-ethyl-N'-(3-carboxy-1-propyl)-2-aminoethyl]-p-toluamid e and 40 mg of bovine serum albumin by the method Example III.

Example VII: p-Benzyloxybenzoic Acid Methyl Ester .

p-Hydroxybenzoic acid methyl ester (1.0 g) was taken up in dry THF (6 ml) and this solution was added dropwise to sodium hydride (0.26 g) suspended in 6 ml THF. After 10 minutes DMF was added dropwise until the solution was clear and benzyl biomide (0.78 g) was added dropwise. Stirring was continued overnight at room temperature. Solvents were removed and the residue was dissolved in $H_2O$ and extracted with $CH_2Cl_2$. The organic layer was washed with $NaHCO_3$ solution and dried over magnesium sulfate. The solvent was removed by rotary evaporation to yield pure product. Yield: 2.79 g.

M.P.: 92-94°C

Example VIII: p-Benzyloxybenzoic Acid.

p-Benxyloxybenzoic acid methyl ester was taken up in 9 ml MeOH and 9 ml of 1N NaOH. The solution was refluxed for 3 hours, cooled and poured into excess $H_20$. Hydrochloric acid was added until pH was acidic, and the solution was extracted with dichloromethane. The organic layer was dried over magnesium sulfate and the solvent removed to yield the product as white needles. Yield: 2.1 g.

Example IX: N-[N'-Ethyl-(2-aminoethyl)]-p-benzyloxybenzamide.

p-Benzyloxybenzoic acid (3.42 g) was taken up in 10 ml dichloromethane and DMF was added dropwise until all material was in solution. N-hydroxysuccinimide (1.72 g) and dicyclohexycar-

bodiimide (3.09 g) were added. The reaction mixture was stirred for one hour at room temperature and filtered and N-ethyl ethylenediamine (1.32 g) was added. After stirring for 30 minutes at room temperature the solvents were removed and the residue was placed under high vacuum overnight.

Example X: N-[N'-Ethyl-N'-(3-carboethoxy-1-propyl)-2-aminoethyl]-p-benzyloxy-benzamide .

N-[N'-Ethyl-2-aminoethyl]-p-benzyloxybenzamide was taken up in 4 ml DMF. Ethylbromocrotonate (0.36 g) was added and the reaction was stirred for 30 minutes at room temperature. The solvent was removed by rotary evaporation and the residue was dissolved in 10 ml dichloromethane. The organic layer was washed 3 times with 10 ml brine, dried over magnesium sulfate and the solvent was removed to yield crude product. It was purified by column chromatography on silica gel, eluting with dichloromethane-methanol. Yield: 0.42 g.

Example XI: N-[N'-Ethyl-N'-(3-carboethoxy-1-propyl)]-2-aminoethyl]-p-hydroxybenzamide

N-[N'-Ethyl-N'-(3-carboethoxy-1-propyl)]-2-aminoethyl-p-benzyloxybenzamide (140 mg) was taken up in 5.0 ml of 95% EtOH and 20 mg of 10% Pd on carbon was added. Hydrogenation was carried out on a low pressure hydrogenator at 25 psi for one hour. The solution was filtered and the filtrate was concentrated and dried under vacuum to yield product. Yield: 27 mg.

Example XII: N-[N'-Ethyl-N'-(3-carboxy-1-propyl) - 2-aminoethyl]-p-hydroxybenzamide .

N-[N'-Ethyl-N'-(3-carboxy-1-propyl)-2-aminoethyl] -p-hydroxybenzamide (17 mg) was taken up in 0.5 ml ethanol and 1.0 ml of 1.0 M sodium hydroxide was added. After stirring for one hour at room temperature hydrochloric acid was added dropwise to acidic pH. Solvent was removed by rotary evaporation and the product was purified on a reversed-phase prep plate, eluting with 50/50 water-methanol containing 0.5% acetic acid. Yield: 12.4 mg.

Example XIII: N-[N'-Ethyl-2-aminoethyl]benzamide.

Methyl benzoate (6.80 g) and N-ethyl ethylenediamine (6.60 g) were dissolved in 209 mL of toluene and heated under reflux for 2 1/2 days. The solvent was removed, and the residue was partitioned between dilute hydrochloric acid and dichloromethane. The aqueous layer was basified with solid potassium hydroxide and extracted twice with dichloromethane. After drying over potassium carbonate, removal of the solvent left 8.72 g of the product as an oil.

Example XIV: N-[N'-Ethyl, N'-(3-ethoxycarbonyl-prop-2-en-1-yl)-2-aminoethyl]benzamide.

N-(N'-Ethyl)-2-aminoethyl benzamide (2.69 g) and ethylbromocrotonate (2.97 g) were allowed to stand for 2 hr at room temperature in 25 mL of dimethylformamide. Potassium carbonate (3.86 g) was added and the mixture was stirred at room temperature overnight. The solvent was removed, add the residue was taken up is dichloromethane, filtered to remove inorganic salts, washed with brine, and dried over sodium sulfate. Removal of solvent left 4.86 g of an oil, which was purified by chromatography over 50 g of neutral alumina, eluting with dichloromethane, to give 3.02 g of the product as a clear oil.

Example XV: N-[N'-Ethyl, N"-(3-ethoxycarbonyl-1-propyl)-2-aminoethyl)benzamide.

N-[N'-Ethyl, N'-(3-ethoxycarbonyl-prop-2-en-1-yl)-2-aminoethyl]benzamide (1.0 g) was dissolved in 50 mL ethanol and hydrogenated over Raney nickel (Aldrich Chemical Company) at room temperature and an initial pressure of 3 atmospheres. Product was isolated by filtration and removal of solvent to give 0.97 g of an oil.

Example XVI: N-[N'-Ethyl, N'-(3-carboxy-1-propyl)-2-aminoethyl]benzamide.

N-[N'-Ethyl, N'-(3-ethoxycarbonyl-1-propyl)-2-aminoethyl]benzamide (492 mg, 1.6 mmol) was dissolved in 5 mL of methanol and treated with 3.2 mL of 1.0M NaOH solution. After standing at room temperature for 5 hr, 530 mL of 3M HCl (1.6 mmol) was added and the solvents were removed. After stripping several times with acetonitrite to remove residual water, the residue was taken up in methanol and filtered from sodium chloride. Removal of solvent left the product as a colorless gum.

Example XVII: N-[(N'-Ethyl)-2-aminoethyl]-p-fluorobenzamide.

N-Ethylethylenediamine (1.76 g) was dissolved in 20 mL of dichloromethane and chilled in an ice-salt bath. A similarly chilled solution of p-fluorobenzyl chloride (1.59 g) in 20 mL of dichloromethane

was added dropwise with good stirring over a 30 min period. The mixture was allowed to warm to room temperature for 10 minutes and the solvent was removed. The residue was taken up in dilute hydrochloric acid, extracted with dichloromethane, basified with solid sodium hydroxide, extracted into dichloromethane, dried over potassium carbonate, filtered and concentrated to leave 1.01 g of the title compound as a colorless oil.

Example XIX: N-(N'-Ethyl, N'-cyanomethyl-2-aminoethyl)-p-fluorobenzamide.

N-(N'-Ethyl-2-aminoethyl)-p-fluorobenzamide - (654 mg), triethylamine (650 mL) and bromoacetonitrile (559 mg) were dissolved in 2 mL of methanol and allowed to stand at room temperature for 2 hours. The solvent was removed, and the residue dissolved in dilute hydrochloric acid. After extraction with ether, the aqueous layer was basified with solid potassium hydroxide and extracted with dichloromethane. After removal of solvent, the residue was purified by chromatography over 40 g of silica gel with dichloromethane-methanol to give 626 mg of a clear oil.

Example XX: N-[N'-Ethyl-(2-aminoethyl)-2-aminoethyl]p-fluorobenzamide.

N-(N'-Ethyl-2-aminoethyl)p-fluorobenzamide - (0.34 g) was hydrogenated overnight ovér 400 mg of #30 Raney nickel in 100 mL of 90:10 methanol-ammonia under an initial pressure of 3 atmospheres. Filtration from catalyst and removal of solvent left 0.35 g of a nearly colorless oil.

Example XXI: N-[N'-(2-Propyl)-2-aminoethyl]-benzamide .

Benzoyl chloride (2.11 g) was dissolved in 5.5 mL of 2,2,2-trifluoroethanol, capped and allowed to stand overnight at room temperature, then heated briefly to reflux. Volatile materials were removed and the residue was again taken up in 2 mL of trifluoroethanol and heated briefly to reflux temperature. All volatile materials were again removed. The residue was dissolved in 10 mL of dichloromethane, chilled in an ice bath and 1.84 of N-isopropylethylenediamine was added over a 5 min period. The' mixtrue was allowed to stand in a refrigerator for 2 1/2 days. Volatile materials were removed and the residue partitioned between dilute hydrochloric acid and ether. Basification of the aqueous layer with solid potassium hydroxide, extraction with ether, drying and removal of solvent left 2.25 g of the product as a colorless oil.

Example XXII: N-[(N'-Ethyl )-2-aminoethyl]-p-toluamide.

This compound was prepared from 3.09 g of p-toluoyl chloride and 1.94 g of N-ethylethylenediamine, employing excess 2,2,2-trifluoroethanol according to the method of Example XXI. Yield: 3.00 g.

Example XXIII: N-[(N'-Ethyl)-2-aminoethyl]-p-chlorobenzamide.

This compound was prepared from 8.75 g of p-chlorobenzoyl chloride and 5.51 g of N-ethyl ethylenediamine according to the method of Example XVII. Yield: 5.09 g.

Example XXIV: N-[N'-(2-Propyl)-2-aminoethyl]-p-nitrobenzamide.

This compound was prepared from 1.856 g of p-nitrobenzoyl chloride and 1.022 g of N-isopropyl ethylenediamine according to the method of Example XVII, except that here the product crystallized from the reaction mixture and was isolated by filtration. Yield: 2.46 g of the hydrochloride.

Example XXV: N-[N'(2-Propyl), N'-(3-ethoxycarbonylprop-2-en-1-yl)-2-aminoethyl]benzamide.

This compound was prepared by the method of Example XIV. From 2.18 g of N-[N'-(2-propyl)-2-aminoethyl]benzamide and 2.25 g of ethyl 4-bromocrotonate were obtained 2.07 g of the title compound as a pale yellow oil.

Example XXVI: N-[N'-Ethyl, N'(3-ethoxycarbonylprop-2-en-1-yl)-2-aminoethyl] p-fluorobenzamide.

This compound was prepared from 8.1 g of N-[N'-ethyl-2-aminoethyl] p-fluorobenzamide and 7.44 g of ethyl 4-bromocrotonate according to the method of Example XIV. The yield was 4.0 g of purified product.

Example XXVII: N-[N'-Ethyl, N'(3-ethoxycarbonylprop-2-en-1-yl)-2-aminoethyl] p-toluamide.

This compound was prepared from 3.43 g of N-[N'-ethyl-2-aminoethyl] p-toluamide and 3.53 g of ethyl 4-bromocrotonate according to the procedure of Example XIV. The purified product weighed 3.64 g.

Example XXVIII: N-[N'-Ethyl, N'-(3-ethoxycarbonyl-prop-2-en-1-yl)-2-aminoethyl]-p-chlorobenzamide.

From 3.38 g of N-[N'-ethyl-2-aminoethyl] p-chlorobenzamide and 3.18 g of ethyl 4-bromocrotonate were obtained 3.47 g of purified title compound according to the procedure of Example XIV.

Example XIX: N-[N'-(2-Propyl), N'-(3-ethoxycarbonyl-1-propyl)-2-aminoethyl] benzamide.

When the procedure of Example XV was carried out with 2.02 g of N-N'-(2-propyl), N'-(3-ethoxy-carbonylprop-2-en-l-yl)-2-aminoethyl] benzamide, 1.93 g of the title compound were obtained as a nearly colorless oil.

Example XXX: N-[N'-Ethyl, N'-(3-ethoxycarbonylprop-2-en-l-yl)-2-aminoethyl] p-fluorobenzamide.

N-[N'-Ethyl, N'-(3-ethoxycarbonylprop-2-en-1-yl)-2-aminoethyl] p-fluorobenzamide (4.0 g) was hydrogenated over 400 mg of 10% palladium or charcoal in ethanol at 3 atmostpheres pressure. Filtration from catalyst and removal of solvent left 3.89 g of an oil.

Example XXXI: N-[N'-Ethyl, N'-(3-ethoxycarbonyl-1-propyl)-2-aminoethyl] p-toluamide.

This compound was prepared by the method of Example XV. N-[N'-Ethyl, N'(3-ethoxycarbonylprop-2-en-1-yl)-2-aminoethyl] p-toluamide (3.55 g) yielded 3.29 g of the title compound as a nearly colorless oil.

Example XXXII: N'[N'-Ethyl, N'-(3-ethoxycarbonyl-1-propyl)-2-aminoethyl] p-chlorobenzamide.

This compound was prepared by the method of Example XV from 1.40 g of N-N'-ethyl, N'-(3-ethoxycarbonyl-prop-2-en-1-yl)-2-aminoethyl] p-chlorobenzamide, except that the crude product was contaminated by a small amount of an impurity and required chromatography purification. The final yield was 663 mg.

Example XXXIII: N-[N'-(2-Propyl), N'-(3-carboxy-1-propyl)-2-aminoethyl] benzamide

This compound was prepared from 1.80 g of N-[N'-(2-propyl), N'-(3-ethoxycarbonyl-1-propyl)-2-aminoethyl] benzamide according to the procedure of Example XVI.

Example XXXIV: N-[N'-Ethyl, N'-(3-carboxy-1-propyl)-2-aminoethyl] p-fluorobenzamide

This compound was prepared from 3.89 g of N-[N'ethyl , N'-(3-ethoxycarbonyl-1-propyl)-2-aminoethyl] p-fluorobenzamide and 0.48 g of sodium hydroxide in ethanol-water. After 3 hr at room temperature, the solution was made acidic with dilute hydrochloric acid extraction of the product with dichloromethane, drying over magnesium sulfate and removal of solvent gave a yield of 2.0 g.

Example XXXV: N-[N'-Ethyl-(3-carboxy-1-propyl)-2-aminoethyl]p-toluamide.

This compound was prepared from 3.02 g of N-[N'-ethyl, N'-(3-ethoxycarbonyl-1-propyl)-2-amino-ethyl] p-toluamide by the method of Example XVI. The portion of the material used for preparation of the immunogen was purified by chromatography on a thick layer plate, developing with 2:1 chloroform-methanol.

Example XXXVI: N-[N'-Ethyl, N'-(3-carboxy-1-propyl)-2-aminoethyl] p-chlorobenzamide.

This compound was prepared from 99 mg of N-[N'-ethyl, N'-(3-ethoxycabonyl-1-propyl)-2-aminoethyl] p-chlorobenzamide by the method of Example XVI. The yield was 87 mg of a nearly colorless gum.

Example XXXVII: N-(N'-Ethyl, N'-cyanomethyl-2-aminoethyl) benzamide .

This compound was prepared according to the procedure of Example XIX from 1.92 g of N-(N'-ethyl-2-aminoethyl) benzamide and 2.4 g of bromoacetonitrile. The yield after chromatography was 1.29 g.

Example XXXVIII: N-[N'-Ethyl-N'-(cyanomethyl)-2-aminoethyl]-p-nitrobenzamide.

This compound was prepared from 237 mg of N-(N'ethyl-2-aminoethyl)-p-nitrobenzamide and 240 mg of bromoacetonitrile according to the procedure of Example XIX except that diisopropyl ethylamine was used as the base instead of triethylamine and the reaction time was increased to 24 hrs. The pale yellow crystalline product melted at 115 to 116°C.

Example XXXIX: N-[N'-Ethyl, N'-(2-cyanoethyl)-2-aminoethyl]benzamide.

This compound was prepared from 960 mg of N-(N'-ethyl-2-aminoethyl)benzamide and 1.79 g 3-bromopropionitrile by the procedure of Example XIX, except that diisopropyl ethylamine was used as the base instead of triethylamine. After some mechanical losses, the yield was 265 mg.

Example XL: N-[N-Ethyl, N-(cyanoethyl)-2-aminoethyl]-p-nitrobenzamide.

N-(N'-Ethyl-2-aminoethyl)-p-nitrobenzamide - (1.37 g), 3-bromopropionitrile (1.00 g) and potassium carbonate (1.38 g) in 6 mL of dimethylformamide were stirred at room temperature overnight. An additional 0.34 g of 3-bromopropionitrile was added, and the mixture was heated in a 50 to 60° oil bath for 7 hr. The mixture was diluted with dichloromethane and filtered to remove inorganic salts and the solvents were removed. The residue was chromatographed over 30 g of silica gel, eluting with dichloromethane-methanol, to give 1.38 g of the product.

Example XLI: N-[N'-Ethyl, N'-(2-cyano ethyl)-2-aminoethyl]-p-fluorobenzamide

N-[N'-Ethyl-2-aminoethyl)-p-fluorobenzamide - (210 mg) in 2 mL of tetrahydrofuran was treated with 64 mg of acrylonitrile and 0.025 mL of 2M sodium hydroxide and stirred at room temperature overnight. The temperature was raised to 55°C, and additional acrylonitrile (127 mg amounts) was added after 4 and after 20 hours. The total time at 55 to 60°C was 24 hr. The product was isolated by passing the crude material through 2.5 g of silica gel, eluting with chloroform-methanol, to give 265 mg of a clear oil.

Example XLII: N-[N'-Ethyl, N-(3-cyano-1-propyl)-2-aminoethyl]-p-fluorobenzamide.

This compound was prepared from 210 mg of N-(N'ethyl-2-aminoethyl)-p-fluorobenzamide and 163 mg of 4-bromobutyronitrile by the procedure of Example XIX, except that no triethylamine base was added. The yield of the title compound after thick layer chromatography was 105 mg.

Example XLIII: N-[N'-Ethyl, N'-(2-aminoethyl)-2-aminoethyl] benzamide.

This compound was prepared from 455 mg of N-[N'-ethyl, N'-cyanomethyl-2-aminoethyl] benzamide according to the procedure of Example XX. The yield of product was slightly greater than the theoretical because it contained some nickel salts. These did not interfere with subsequent reactions.

Example XLIV: N-[N'-Ethyl, N'-(2-aminoethyl)-2-aminoethyl]-p-aminobenzamide.

This compound was prepared from 92 mg of N-[N'-ethyl, N'-(cyanomethyl)-2-aminoethyl]-p-nitrobenzamide according to the procedure of Example XX except that 5% rhodium on carbon was used as the catalyst instead of Raney nickel. The product, which weighed 62 mg, was not as clean as those produced in Raney nickel reductions.

Example XLV: N-[N'-Ethyl, N'-(3-amino-1-propyl)-2-aminoethyl]-benzamide.

This compound was prepared from 239 mg of N-[N'-ethyl, N'-(2-cyanoethyl)-2-aminoethyl] benzamide according to the procedure of Example XX. Yield: 205 mg of pale green oil.

Example XLVI: N-[N'-Ethyl, N'-(3-amino-1-propyl)-2-aminoethyl]-p-fluorobenzamide.

This compound was prepared from 200 mg of N[N-ethyl, N'-(2-cyanoethyl)-2-aminoethyl]-p-fluorobenzamide according to the procedure of Example XX.

Example XLVII: N-[N'-Ethyl, N'-(4-amino-1-butyl)-2-aminoethyl]-p-fluorobenzamide.

This compound was prepared from 100 mg of N-[N'-ethyl, N'-(3-cyano-1-propyl)-2-amino-ethyl]-p-fluorobenzamide according to the procedure of Example XX.

Example XLVIII: 5-(2-aminoethylaminocarboxy)-fluoroescein.

5-Carboxyfluorescein N-hydroxysuccinimidyl ester (500 mg) was taken up in 2 mL of dimethylformamide and added to a solutio of 635 mg of ethylenedianime in 2 ml of dimethylformamide. The mixture was stirred at room temperature for 30 minutes and diluted with a large volume of ethyl ether, and the precipitate was collected by filtration.

Example XLIX: N-[N'-Ethyl, N'-(fluorescein-5-ylaminocarbonylmethyl)-2-aminoethyl] benzamide.

N-(N'-Ethyl-2-aminoethyl) benzamide (25 mg, 0.1 mmol), 5-bromoacetamidofluorescein (23 mg, 0.05 mmol) and triethylamine (17.7 mg, 0.175 mmol) were dissolved in 0.25 mL of methanol and stirred at room temperature overnight. The product was isolated by chromatography on a silica gel thick layer plate, developing with 3:1 chloroform-methanol.

Example L: N-[N'-Ethyl, N'-(fluorescein-5-ylaminocarbonylmethyl)-2-aminoethyl]p-fluorobenzamide.

N-(N'-Ethyl-2-aminoethyl)-p-fluorobenzamide hydrochloride (25 mg., 0.1 mmol), 5-bromoacetamidofluorescein (23 mg., 0.05 mmol) and triethylamine (0.026 ml, 0.15 mmol) were dissolved in 0.25 ml of methanol and stirred at room temperature for 64 hours. The product was isolated by chromatography on a silica gel thick layer plate developed with 3:1 chloroform-methanol.

Example LI: N-[N'-Ethyl, N'-(fluorescein-5-ylaminocarbonyl methyl)-2-aminoethyl]4-toluamide.

N-(N'-Ethyl-2-aminoethyl)4-toluamide (12 mg, 0.05 mmol), 5-bromoacetamidofluorescein (23 mg, 0.05 mmol) annd triethylamine (12.5 mg, 0.125 mmol) were dissolved in 0.25 ml of methanol and allowed to stand at room temperature for 36 hours. Chromatography on a thick layer silica gel plate with 2:1 chloroform-methanol afforded the pure product.

Example LII: N-[N'-Ethyl, N'-(fluorescein-5-ylaminocarbonyl-methyl)-2-aminoethyl]-p-chlorobenzamide

N-[N'-Ethyl-2-aminoethyl)-p-chlorobenzamide - (20 mg, 0.088 mmol), 5-bromoacetamidofluorescein (23 mg, 0.05 mmol) and triethylamine (0.0105 ml, 0.075 mmol) were dissolved in 0.25 ml of methanol and allowed to stand at room temperature for 24 hours. The product was isolated by chromatography on silica gel thick layer plates, developed with 3:1 chloroform-methanol.

Example LIII: N-[N'-(2-Propyl), N'-(fluorescein-5-yl-aminocarbonylmethyl)-2-aminoethyl]-p-aminobenzamide

N-[N'-2-Propyl)-2-aminoethyl]-p-nitrobenzamide (29 mg) and 5-bromoacetamidofluorescein (23 mg) were taken up in 0.5 ml of methanol and stirred at room temperature for 36 hours. The product was isolated by chromatography on a silica gel thick layer plate, eluting with chloroform-methanol. It was taken up in 7 ml of methanol and 2 ml of 15% aqueous potassium hydroxide and reduced by the addition of 1 ml of 10% aqueous solution of ferrous sulfate heptahydrate. The resulting precipitate was removed by centrifugation, pH was adjusted to 6.7 and solvents were removed. A second chromatography on a silica gel plate with chloroform-methanol gave the pure product.

Example LIV: N-[N'-Ethyl, N'-(fluorescein-5-yl-aminocarbonylmethyl)-2-aminoethyl]-p-aminobenzamide

This compound was prepared according to the method of Example LIII, starting with 40 mg of N-[N'-ethyl-)-2-aminoethyl]-p-nitrobenzamide and 94 mg of 5-bromoacetamidofluorescein.

Example LV: N-[N'-Ethyl,N-(3-fluorescein-6-ylaminocarbonyl-1-propyl)2-aminoethyl]benzamide

N-[N'-Ethyl,N'-(3-carboxy-1-propyl)-2-aminoethyl] benzamide (17.8 mg, 0.064 mmol) was dissolved in 0.10 ml dry dimmethylformamide, chilled in an ice bath and 13.2 mg (0.01 mmol) of isobutylchloroformate was added. The solution was stirred for 3 hours as the ice melted and it warmed up to room temperature. Half of this mixture was then added to 11 mg. (0.032 mmol) of 6-aminofluorescein, and 0.05 ml of pyridine was added. After stirring at room temperature overnight, the product was isolated by chromatography on a silica gel thick layer plate, developing with 3:1 chloroform-methanol.

Example LVI: N-[N'-(2-Propyl),N-(3-fluorescein-5-ylaminocarbonyl-1-propyl)-2-aminoethyl]benzamide

This compound was prepared by the method of Example LV from 63 mg of N-[N'-(2-propyl),N'-(3-carboxy-1-propyl)-2-aminoethyl]benzamide, 41 mg of isobutyl chloroformate and 28 mg of 5-aminofluorescein. Chloroform-methanol (2:1) was used for the chromatographic purification.

Example LVII: N-[N'-Ethyl,N'-(3-fluorescein-6-yl-aminocarbonyl-1-propyl)-2-aminoethyl]-p-chlorobenzamide

N-[N'-Ethyl,N'-(3-carboxy-1-propyl)-2-aminoethyl]-p -chlorobenzamide (87 mg) and 40.5 mg of carbonyldiimidazole were taken up in 2 ml of acetonitrile. After stirring for 1 1/2 hour at room temperature, a 0.40 ml aliquot was removed, concentrated on a rotary evaporator, redissolved in 0.10 ml of dimethylformamide and allowed to react with 17.4 mg of 6-aminofluorescein at room temperature for 4 1/2 days. The product was purified by chromatographing twice on thin layer plates with 3:1 chloroform-methanol.

Example LVIII: N-[N'-Ethyl,N'-{3-(fluorescein-5-ylcarbonylamino-2-ethylaminocarbonyl)-1-propyl}-2-aminoethyl]-p-hydroxybenzamide

N-[N'-Ethyl,N'-(3-carboxy-1-propyl)]-2-aminoethyl-p-hydroxybenzamide (80 mg) was taken up in 2 ml of dimethylformamide and N-hydroxysuccinimide (41 mg) and dicyclohexylcarbodimide (73 mg) were added. The solution was stirred overnight at room temperature and filtered into a solution of 5-(2-aminoethylaminocarboxy)fluorescein - (114 mg) and triethylamine (28 mg) in 1 ml dimethylformamide. The mixture was again stirred overnight at room temperature, and the solvent was removed. The product was purified by chromatography on a reversed-phase chromatography plate.

Example LIX: N-[N'-Ethyl,N'-2-{(fluorescein-6-ylcarbonyl)-amino}-1-ethyl]benzamide

N-[N'-Ethyl,N'-(2-amino-1-ethyl)-2-aminoethyl]-benza mide (30 mg) was allowed to react with a solution of activated 6-carboxyfluorescein formed from 31 mg of 6-carboxyfluorescein, 13.9 mg of 1-hydroxybenzotriazole hydrate and 48.2 mg of dicyclohexylcarbodiimide which had been allowed to stir in 0.25 ml of pyridine for 2 hours beforehand. The final mixture was stirred overnight, and the product was isolated by chromatography, first on a silicagel thick layer plate with chloroform-methanol, then on a reversed phase plate with methanol-ammonium acetate buffer.

Example LX: N-[N'-Ethyl,N'-(2-{ 4-(fluorescein-6-ylamino)-6-chloro-1,3,5,-triazin-2-yllamino}-1-ethyl)-]benzamide

N-[N'-Ethyl,N'-(2-aminoethyl)-2-aminoethyl] benzamide (8 mg) and 6-[(4,6-dichloro-1,3,5-triazin-2-yl) amino]fluorescein (17.5 mg) were dissolved in 0.3 ml of methanol. Triethylamine (0.015 ml) was added, and the mixture was stirred at room temperature for 3 hours. The product was purified by preparative thin layer chromatography, developing with chloroform-methanol.

Example LXI: N-[N'-Ethyl,N'-(2-{ 4-(fluorescein-5-ylamino)-6-chloro-1,3,5-triazin-2-ylamino}-1-ethyl)] benzamide

This compound was prepared according to the method of Example LX from 13 mg of N-[N'-ethyl,N'-(2-aminoethyl)-2-aminoethyl]benzamide and 29 mg of 5-[4,6-dichloro-1,3,5-triazin-2-yl)-amino] fluorescein.

Example LXII: N-[N'-Ethyl,N'-(2-{4-(fluorescein-5-ylamino)-6-chloro-1,3,5-triazin-2-ylamino}-1-ethyl)]-p-fluorobenzamide

This compound was prepared according to the method of Example LX from 9 mg of N-[N'-ethyl,N'-(2-aminoethyl)-2-aminoethyl]benzamide and 19.1 mg of 5-[(4,6-dichloro-1,3,5-triazin-2-yl)-amino] fluorescein.

Example LXIII: N-[N'-Ethyl,N'-(2-{4-(fluorescein-5-yl amino)-6-chloro,1,3,5-triazin-2-ylamino}-1-ethyl)]-p-aminobenzamide

This compound was prepared according to the method of Example LX except that the reaction was initiated in an ice bath and allowed to warm to room temperature gradually over 3 1/2 hr. Starting materials were 15 mg of N-[N'-ethyl,N'-(2-aminoethyl)-2-aminoethyl]benzamide and 22.7 mg of 5-[(4,6-dichloro-1,3,5-triazin-2-yl)amino] fluorescein. Chromatography solvents were ethyl acetate-methanol and then chloroform-methanol.

Example LXIV: N-[N'-Ethyl,N'-(3-{ 4-(fluorescein-5-ylamino)-6-chloro-1,3,5-triazin-2-ylamino}-1-propyl)] benzamide

This compound was prepared according to the method of Example LX from 7 mg of N-[N'-ethyl,N'-(3-amino-1-propyl)-2-aminoethyl]-benzamide and 14.9 mg of 5-[(4,6-dichloro-1,3,5-triazin-2-yl) amino] fluorescein.

Example LXV: N-[N'-Ethyl,N'-(3-{4-(fluorescein-5-ylamino)-6-chloro-1,3,5-triazin-2-ylamino }-1-propyl)]benzamide

This compound was prepared according to the method of Example LX from 6.2 mg of N-[N'-ethyl,N'-(3-amino-1-propyl)-2-aminoethyl]-p-fluorobenz amide and 12.6 mg of 5-[(4,6-dichloro-1,3,5-triazin-2-yl) amino]fluorescein.

Example LXVI: N-[N'-Ethyl,N'-(4-{4-(fluorescein-5-ylamino)-6-chloro-1,3,5-triazin-2-ylamino}-1-butyl)]-p-fluorobenzamide

Ths compound was made according to the method of Example LX, except that reaction time was 16 hours at 4 degrees C, then 1 hour at room temperature. Starting materials were 5 mg of N-[N'-ethyl,N'-(4-amino-1-butyl)-2-aminoethyl]-p-fluorobenza mide and 9.5 mg of 5-[(4,6-dichloro-1,3,5-triazin-2-yl)amino]fluorescein.

## Claims

1. A compound comprising the structure:

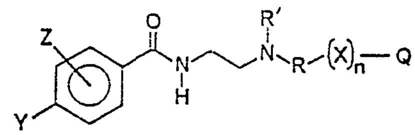

wherein

Q is a poly(amino acid), a poly(amino acid) derivative or another immunologically active carrier, or fluorescein or a fluorescein derivative;

X is NH, CO, CS, C=NH or $SO_2$

n is 0, 1 or 2;

R is a linking group including up to 6 heteroatoms when Q is a poly(amino acid) or a poly(amino acid) derivative or another immunologically active carrier and up to 8 heteroatoms having a total of from 0 to 18 carbon atoms and heteroatoms;

R' is an alkyl radical having from 1 to 3 carbon atoms;

Y is OH, $NH_2$, $CH_3$, F, Cl, Br, $CF_3$ or H when Q is fluorescein or a fluorescein derivative, and Y is H, OH, $CH_3$, F or Cl when Q is a poly(amino acid) or a poly(amino acid) derivative or another immunologically active carrier; and

Z is H or F when Q is fluorescein or a fluorescein derivative, and is H when Q is a poly(amino acid), a poly(amino acid) derivative or another immunologically active carrier; and

wherein Z is attached to any of the carbon atoms of the benzene ring other than those attached to Y or the carboxamide group.

2. The compound of Claim 1 wherein Q is bovine serum albumin.

3. The compound of Claim 1 wherein Q is an amino, an amido, an amidino, a urea, a thiourea, a carbamate, a thiocarbamate, a triazinylamino, or a (carboxyamino)-sulfonamido derivative of fluorescein.

4. The compound of Claim 1 wherein Q is 4-chloro-6-(fluorescein-5-ylamino)-1,3,5-triazin-2-yl.

5. The compound of Claim 1 wherein Q is 4-chloro-6-(fluorescein-6-ylamino)-1,3,5-triazin-2-yl.

6. The compound of Claim 1 wherein Q is 4-methoxy-6-(fluorescein-5-yl-amino)-1,3,5-triazin-2-yl.

7. The compound of Claim 1 wherein Q is 4-methoxy-6-(fluorescein-6-yl-amino)-1,3,5-triazin-2-yl.

8. The compound of claim 1 wherein Q is -(fluorescein-5-yl)amino.

9. The compound of Claim 1 wherein Q is -(fluorescein-6-yl)amino.

10. An antibody to a compound according to Claim 1 wherein Q is a poly(amino acid), a poly(amino acid) derivative or another immunologically active carrier.

11. A method for making an immunogen comprising the step of coupling a precursor of the formula

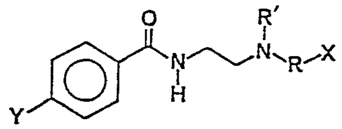

where:

R is a linking group including up to 6 heteroatoms and having a total of from 0 to 18 carbon atoms and heteroatoms;

R' is an alkyl radical having from 1 to 3 carbon atoms;

X is $NH_2$, COOH, CN, CHO, Br, I, or OH; and

Y is H, OH, $CH_3$, F or Cl;

with a poly(amino acid) or a derivative of a poly-(amino acid).

12. The method of Claim 11 wherein the poly-(amino acid) is thyroglobulin.

13. The method of Claim 11 wherein the poly-(amino acid) is bovine serum albumin.

14. The method of Claim 11 wherein the reaction is carried out by active ester coupling.

15. A method for making a tracer comprising the step of coupling a precursor of the formula

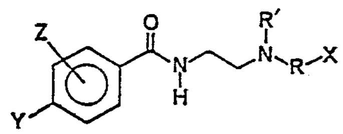

where:

R is a linking group including up to 6 heteroatoms and having a total of from 0 to 18 carbon atoms and heteroatoms;

X is $NH_2$, COH, CN, $SO_3H$ or OH;

Y is OH, $NH_2$, $CH_3$, F, Cl, Br, $CF_3$ or H; and

Z is H or F; with fluorescein or a derivative of fluorescein.

16. A process for measuring concentration of procainamide which comprises the steps of:

(a) contacting a biological sample with a procainamide antiserum and with a compound according to Claim 1 capable of producing a detectable fluorescence polarization response to the presence of the procainamide antiserum;

(b) passing plane polarized light through the resulting solution from step (a) to obtain a fluorescence polarization response; and

(c) detecting the fluorescence polarization response of the solution of step (b) as a measure of the amount of procainamide in the sample.

17. The process of Claim 16 wherein the procainamide antiserum is produced by the antibodies of Claim 10.

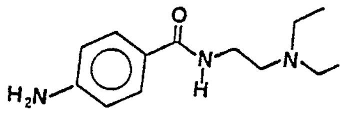

Fig. 1

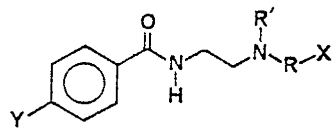

Fig. 2

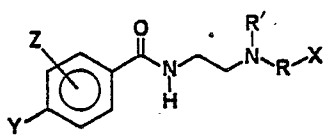

Fig. 3

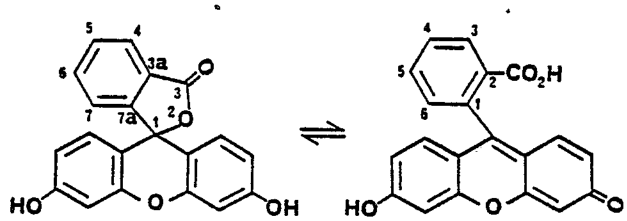

Lactone

Acid

Fig. 4

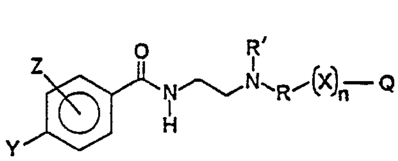

Fig. 5

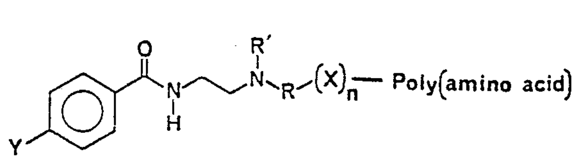

Fig. 6

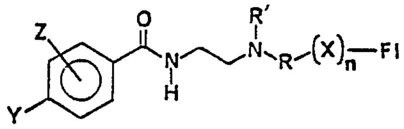

Fig. 7

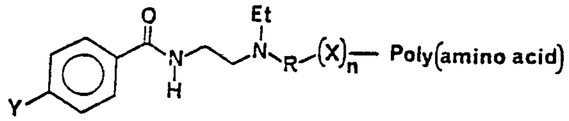

Fig. 8

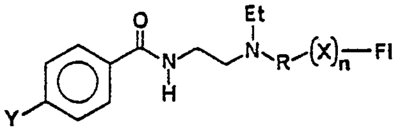

Fig. 9

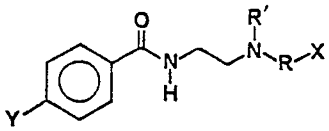

Fig. 10

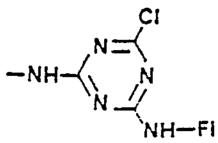

Fig. 11-1

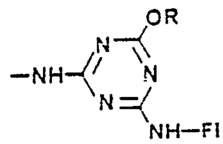

Fig. 11-2

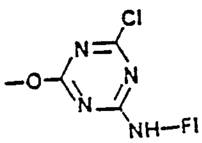

Fig. 11-3

-NH-CO-Fl

Fig. 11-4

-CO-NH-Fl

Fig. 11-5

-CNH-NH-Fl

Fig. 11-6

-NH-CO-NH-Fl

Fig. 11-7

-NH-CS-NH-Fl

Fig. 11-8

-O-CO-NH-Fl

Fig. 11-9

-O-CS-NH-Fl

Fig. 11-10

$-SO_2-NH-Fl$

Fig. 11-11

$-O-CO-NH-SO_2-NH-Fl$

Fig. 11-12

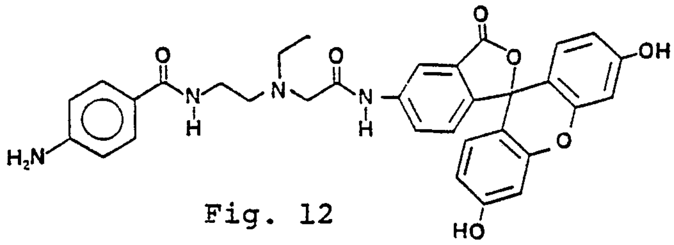

Fig. 12

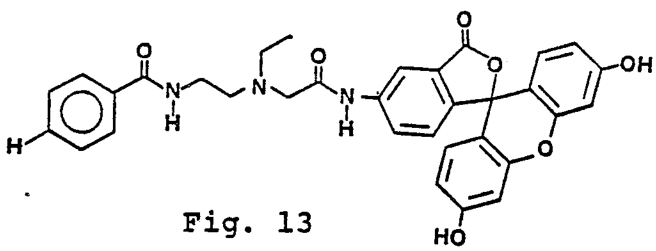

Fig. 13

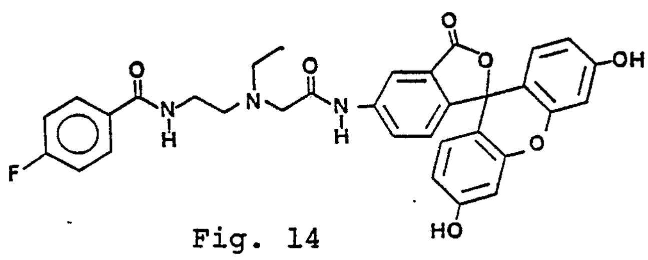

Fig. 14

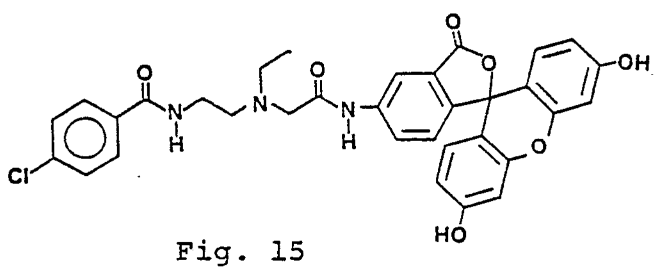

Fig. 15

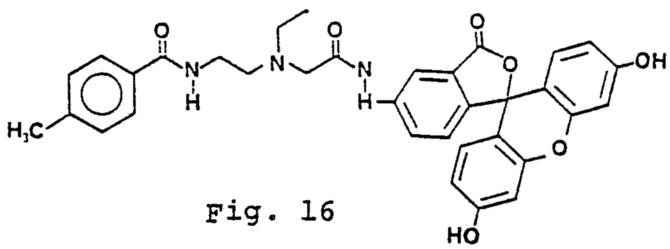

Fig. 16

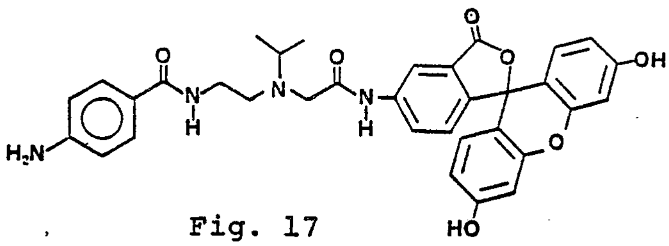

Fig. 17

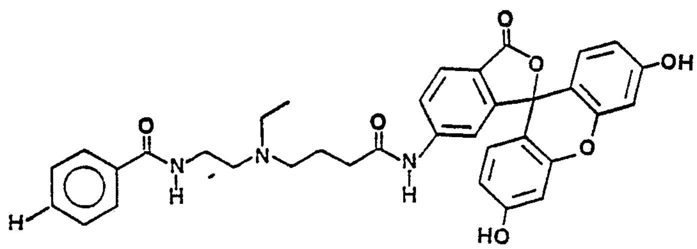

Fig. 18

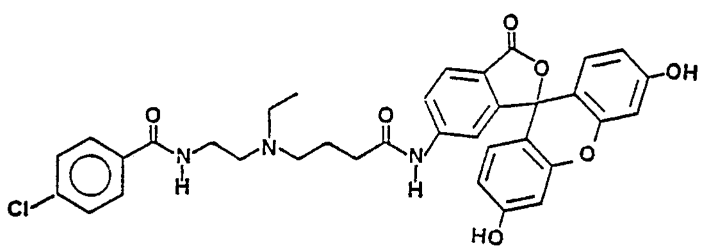

Fig. 19

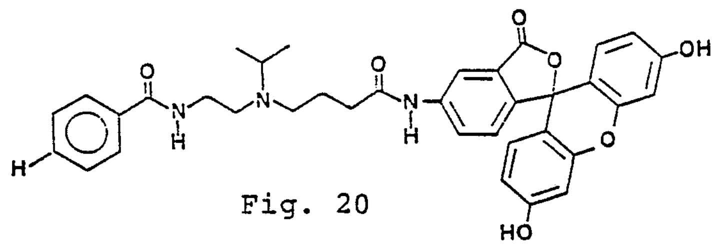

Fig. 20

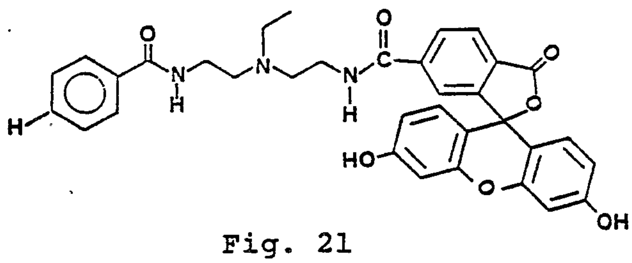

Fig. 21

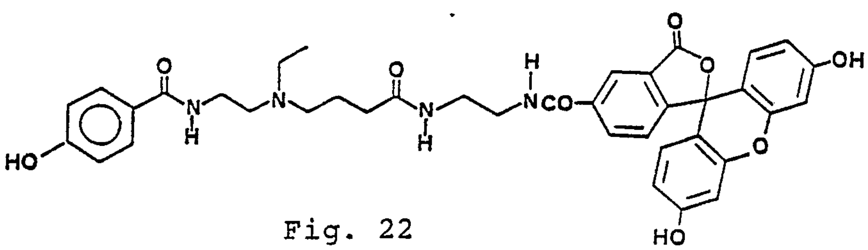

Fig. 22

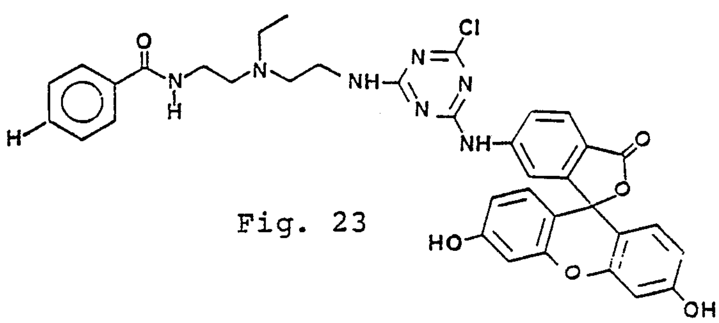

Fig. 23

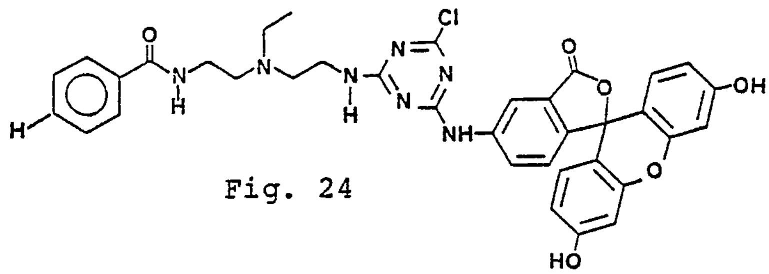

Fig. 24

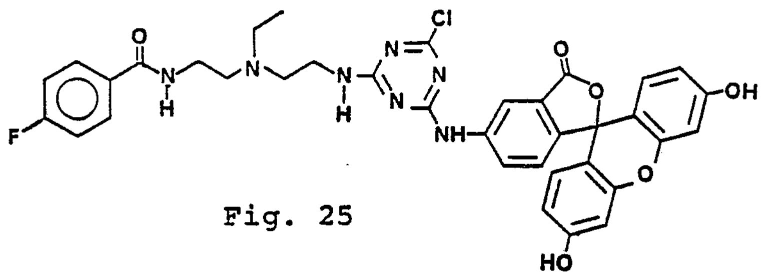

Fig. 25

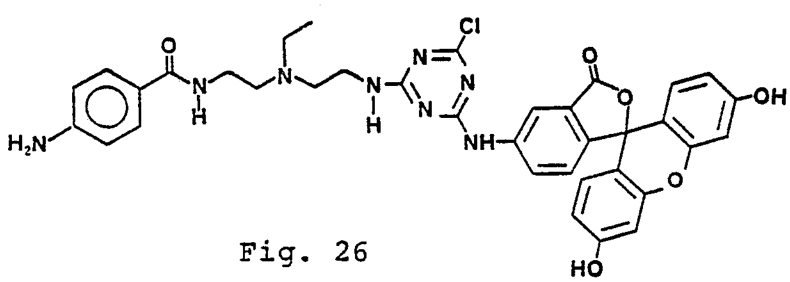

Fig. 26

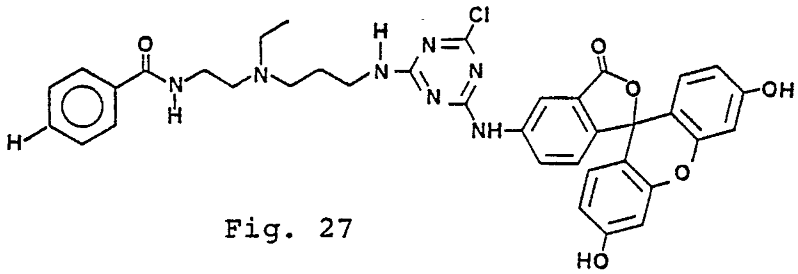

Fig. 27

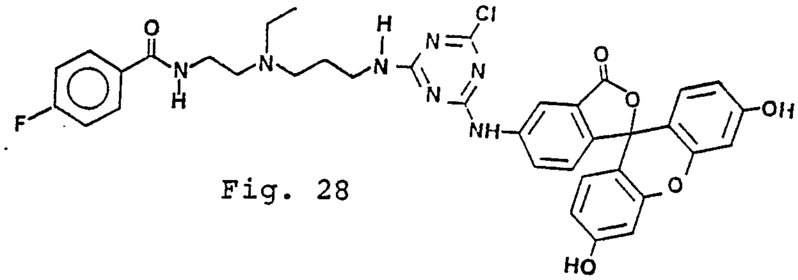

Fig. 28

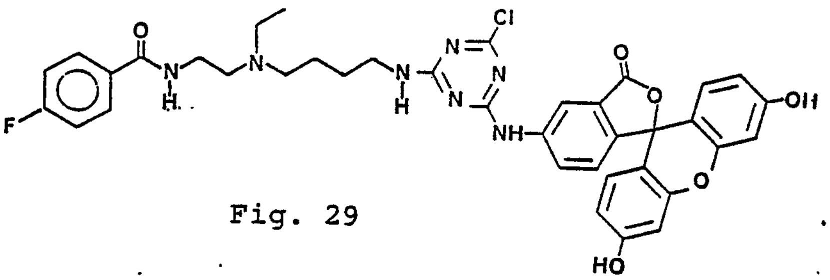

Fig. 29

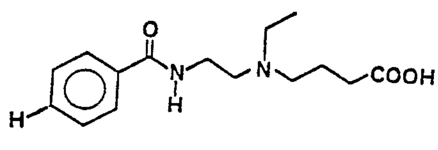

Fig. 30

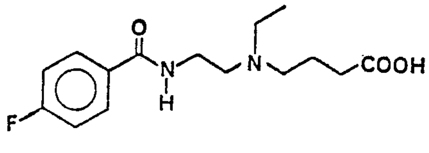

Fig. 31

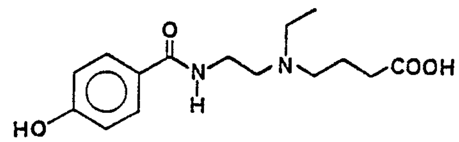

Fig. 32

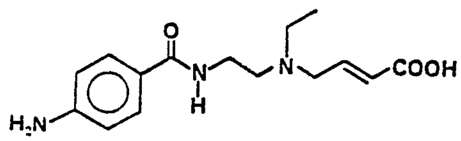

Fig. 33

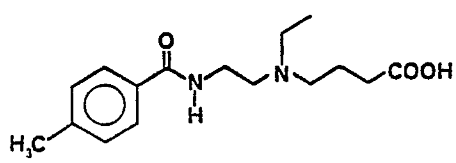

Fig. 34

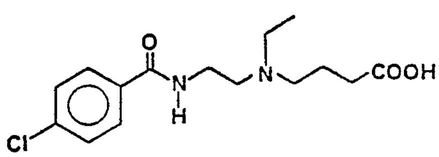

Fig. 35

European Patent Office

# EUROPEAN SEARCH REPORT

Application number

EP 86 10 3161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 500 165 (ABBOT LABORATORIES) * Page 1, line 1 - page 11, line 23; examples 18,19; claims 1-6,13,17-19,36 * | 1,3-5, 15,16 | G 01 N 33/58<br>G 01 N 33/533<br>C 07 D 493/10<br>C 07 K 15/00 |
| A | --- | 14 | |
| Y | GB-A-2 081 257 (ABBOT LABORATORIES) *Abstract; page 1, lines 1-58; examples IV+XXIII; claims 1,7,20 * & US - A - 4 420 568 (Cat. D) --- | 1,3-5, 15,16 | |
| A,D | US-A-4 235 969 (SYVA COMPANY) * Column 1, line 1 - column 4, line 23; claims 1,2,6,7,9,11,12 * --- | 1,2,10 -14 | |
| | | | TECHNICAL FIELDS SEARCHED (Int. Cl.4) |
| A | US-A-4 318 846 (SYVA COMPANY) * Abstract; column 1, line 1 - column 15, line 9; column 21, lines 17-55; claims * --- | 1,3,15 | G 01 N<br>C 07 D |
| A | FR-A-2 518 096 (ABBOT LABORATORIES) * Page 1, line 1 - page 18; line 16, examples 12,13,18; page 20, lines 11-29; claims 1-4,14-16,20-25,32-37,43-47,50-54 * ----- | 1,3,8, 9,15, 16 | |

The present search report has been drawn up for all claims

| Place of search | Date of completion of the search | Examiner |
|---|---|---|
| THE HAGUE | 25-06-1986 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document